# FASCICULE DE BREVET EUROPEEN

(11) **EP 1 248 785 B1**
(45) Date de publication et mention de la délivrance du brevet: **15.06.2005**
(21) Numéro de dépôt: 00990851.8
(22) Date de dépôt: 29.12.2000
(51) Int. Cl.: C07D 409/06, C07D 405/06, A61K 31/4525, A61K 31/4535, A61P 25/28, A61P 25/16, A61P 37/00

(54) **TETRAHYDROPYRIDINES, PROCEDE POUR LEUR PREPARATION ET COMPOSITIONS PHARMACEUTIQUES LES CONTENANT**
TETRAHYDROPYRIDINE, VERFAHREN ZU IHRER HERSTELLUNG UND SIE ENTHALTENDE PHARMAZEUTISCHE ZUBEREITUNGEN
NOVEL TETRAHYDROPYRIDINES, PREPARATION METHOD AND PHARMACEUTICAL COMPOSITIONS CONTAINING SAME

(30) Priorité: 06.01.2000 FR 0000113
(43) Date de publication de la demande: 16.10.2002
(73) Titulaire: Sanofi-Aventis, 75013 Paris (FR)
(72) Inventeur: BARONI, Marco, I-20010 Vanzago (IT); BOURRIE, Bernard, F-34980 Saint Gely du Fesc (FR); CARDAMONE, Rosanna, I-2100 Como (IT); CASELLAS, Pierre, F-34090 Montpellier (FR)
(74) Mandataire: Varady, Peter
(86) Numéro de dépôt international: PCT/FR2000/003741
(87) Numéro de publication internationale: WO 2001/049684

(56) Documents cités:
- WO-A-92/07831
- WO-A-92/19594
- WO-A-97/01536
- WO-A-99/65880
- DE-A- 2 060 816
- GB-A- 2 310 376

## Description

La présente invention concerne de nouvelles tétrahydropyridines, un procédé pour leur préparation et les compositions pharmaceutiques les contenant.

WO92/07831 décrit des tétrahydropyridines substituées par un groupe benzofuran-6-yl-alkyle portant une triple liaison dans la chaîne alkylique, ayant une activité dopaminergique. WO92/19594 décrit des dérivés de pyrrolidinone inhibiteurs de PDE IV (phosphodiesterase) et de TNF (tumor Necrosis Factor).

Il a été maintenant trouvé que certaines tétrahydropyridines, substituées par un radical benzofuryl-alkyle ou benzothiényl-alkyle, possèdent une puissante activité vis-à-vis de la modulation du TNF-alpha (de l'anglais Tumour Necrosis Factor).

Le TNF-alpha est une cytokine qui a récemment suscité de l'intérêt en tant que médiateur de l'immunité, de l'inflammation, de la prolifération cellulaire, de la fibrose etc. Ce médiateur est très élevé dans le tissu synovial inflammé et exerce un rôle important dans la pathogenèse de l'autoimmunité (Annu. Rep. Med. Chem., 1997, 32:241-250).

Ainsi, la présente invention concerne, selon un de ses aspects, des tétrahydropyridines de formule (I) : 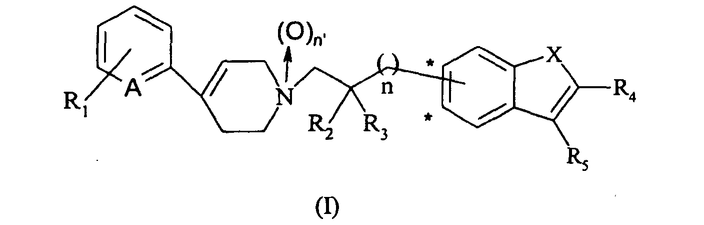 dans laquelle
- R₁: représente un atome d'hydrogène ou d'halogène, ou un groupe CF₃ ;
- R₂ et R₃: représentent indépendamment un atome d'hydrogène ou un groupe méthyle;
- n et n': représentent chacun indépendamment 0 ou 1;
- *: représente les positions de rattachement;
- A: représente N ou CH ;
- X: représente un atome de soufre ou d'oxygène;
- R₄ et R₅: représentent indépendamment un atome d'hydrogène ou un groupe (C₁-C₆) alkyle;
ainsi que leurs sels ou solvates.

Dans la présente description, le terme "(C₁-C₆)alkyle" désigne un radical monovalent d'un hydrocarbure en C₁-C₆ saturé à chaîne droite ou ramifiée.

Dans la présente description, le terme "halogène" désigne un atome choisi parmi le chlore, le brome, le iode et le fluor.

Des composés préférés sont ceux où n est zéro.

D'autres composés préférés sont ceux où R, est dans la position 3 du benzène.

D'autres composés préférés sont ceux où R₁ est un groupe CF₃.

D'autres composés préférés sont ceux où R₂ et R₃ sont chacun un atome d'hydrogène.

D'autres composés préférés sont ceux où R₄ et R₅ sont chacun un groupe méthyle.

Les sels des composés de formule (I) selon la présente invention comprennent aussi bien les sels d'addition avec des acides minéraux ou organiques pharmaceutiquement acceptables tels que le chlorhydrate, le bromhydrate, le sulfate, l'hydrogénosulfate, le dihydrogénophosphate, le citrate, le maléate, le tartrate, le fumarate, le gluconate, le méthanesulfonate, le 2-naphtalènesulfonate, etc., que les sels d'addition qui permettent une séparation ou une cristallisation convenable des composés de formule (I), tels que le picrate, l'oxalate ou les sels d'addition avec des acides optiquement actifs, par exemple les acides camphosulfoniques et les acides mandéliques ou mandéliques substitués.

Les stéréoisomères optiquement purs, ainsi que les mélanges d'isomères des composés de formule (I), dus au carbone asymétrique, lorsque l'un de R₂ et R₃ est un méthyle et l'autre un hydrogène, dans une proportion quelconque, font partie de la présente invention.

Les composés de formule (I) peuvent être synthétisés par un procédé qui prévoit
(a) de faire réagir le composé de formule (II) : 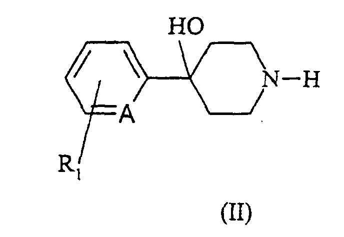 dans laquelle A et R₁ sont définis comme précédemment,
   avec un dérivé fonctionnel de l'acide de formule (III) : 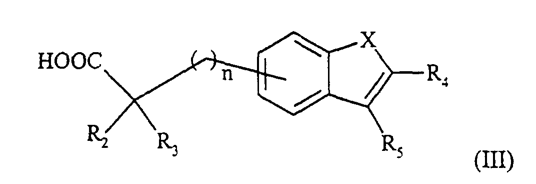 dans laquelle R₂, R₃, R₄, R₅, n et X sont tels que définis ci-dessus,
(b) de réduire le groupe carbonyle du composé de formule (IV) : 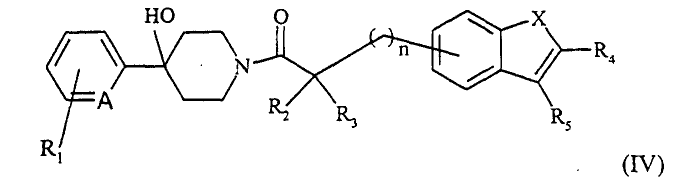
(c) de déshydrater le pipéridinol intermédiaire de formule (V) : 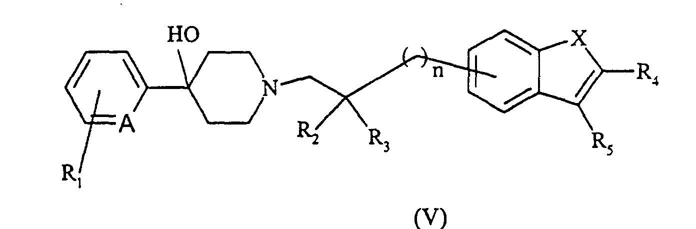
(d) d'isoler le composé de formule (I) ainsi obtenu et, éventuellement, le transformer en l'un de ses sels ou solvates, ou son N-oxyde (n'=1 dans la formule I).

Comme dérivé fonctionnel approprié de l'acide de formule (III), on peut utiliser l'acide libre, éventuellement activé (par exemple avec le BOP = tri(diméthylamino)benzotriazol-1-yloxyphosphonium hexafluorophosphate), un anhydride, un anhydride mixte, un ester actif ou un halogénure d'acide, le bromure de préférence. Parmi les esters actifs, l'ester de p-nitrophényle est particulièrement préféré, mais les esters de méthoxyphényle, de trityle, de benzhydryle et similaires sont également convenables.

La réaction de l'étape (a) peut être convenablement conduite dans un solvant organique à une température comprise entre -10°C et la température de reflux du mélange réactionnel.

Il peut être préférable de réaliser la réaction à froid lorsqu'elle est exothermique, comme dans le cas où on utilise le chlorure en tant que dérivé fonctionnel de l'acide de formule (III).

Comme solvant de réaction, on utilise de préférence un solvant halogéné tel que le chlorure de méthylène, le dichloroéthane, le 1,1,1-trichloroéthane, le chloroforme et similaires, mais aussi d'autres solvants organiques compatibles avec les réactifs employés, par exemple le dioxane, le tétrahydrofurane ou un hydrocarbure tel que l'hexane, peuvent être également employés.

La réaction peut être convenablement conduite en présence d'un accepteur de protons, par exemple d'un carbonate alcalin ou d'une amine tertiaire telle que la triéthylamine.

La réduction de l'étape (b) peut être convenablement réalisée par des agents de réduction appropriés tels que les complexes du borane, par exemple le diméthylsulfure de borane ([CH₃]₂S-BH₃), les hydrures d'aluminium ou un hydrure complexe de lithium et d'aluminium dans un solvant organique inerte à une température comprise entre 0°C et la température de reflux du mélange réactionnel, selon les techniques usuelles.

Par "solvant organique inerte" on entend un solvant qui n'interfère pas avec la réaction. De tels solvants sont par exemple les éthers, tel que l'éther diéthylique, le tétrahydrofurane (THF), le dioxane ou le 1,2-diméthoxyéthane.

Selon un mode opérationnel préférentiel, on opère avec le diméthylsulfure de borane utilisé en excès par rapport au composé (II) de départ, à la température de reflux éventuellement sous atmosphère inerte. La réduction est normalement terminée après quelques heures.

La déshydratation de l'étape (c) est aisément conduite par exemple en utilisant un mélange acide acétique/acide sulfurique, à une température comprise entre la température ambiante et la température de reflux du solvant utilisé ou bien en utilisant l'acide paratoluensulfonique dans un solvant organique, tel que par exemple le benzène, le toluène, le chlorobenzène.

Les composés de formule (I) peuvent aussi être préparés par condensation d'une tétrahydropyridine de formule (VI) : 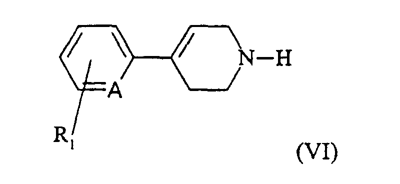 dans laquelle A et R₁ sont tels que définis ci-dessus, avec un composé de formule (VII) : 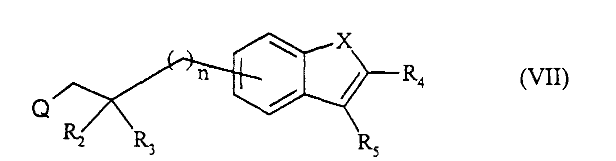 dans laquelle R₂, R₃, R₄, R₅, n et X sont tels que définis ci-dessus et Q est un groupe partant, isolement du composé de formule (I) ainsi obtenu et transformation éventuelle en un de ses sels ou solvates, ou son N-oxyde.

Comme groupe partant Q, on peut utiliser par exemple un atome d'halogène ou tout groupe apte à la condensation avec une amine. La réaction de condensation est réalisée de manière conventionnelle par mélange des composés de départ (VI) et (VII) dans un solvant organique tel qu'un alcool, par exemple le méthanol ou le butanol, en présence d'une base telle qu'un carbonate alcalin, à une température comprise entre la température ambiante et celle de reflux du solvant choisi.

Le composé voulu est isolé selon les techniques conventionnelles sous forme de base libre ou d'un de ses sels. La base libre peut être transformée dans un de ses sels par simple salification dans un solvant organique tel qu'un alcool, de préférence l'éthanol ou l'isopropanol, un éther comme le 1,2-diméthoxyéthane, l'acétate d'éthyle, l'acétone ou un hydrocarbure comme l'hexane. Elle peut être transformée en N-oxyde par oxydation selon des méthodes conventionnelles, par exemple avec de l'acide 3-chloroperbenzoïque.

Les composés de départ de formules (II), (III), (VI) et (VII) sont connus ou bien ils peuvent être préparés de façon analogue aux composés connus.

Les composés de l'invention possèdent des propriétés intéressantes vis-à-vis de l'inhibition du TNF-α.

Ces propriétés ont été mises en évidence à l'aide d'un test visant à mesurer l'effet de molécules sur la synthèse du TNF-α induite chez la souris Balb/c par du lipopolysaccharide (LPS) d'Escherichia Coli (055 :B5, Sigma, St Louis, Mo).

Les produits à tester sont administrés par voie orale à des groupes de 5 souris Balb/c femelles (Charles River, France) âgées de 7 à 8 semaines. Une heure après, le LPS est administré par voie intraveineuse (10µg/souris). Le sang de chaque animal est prélevé 1,5 heure après l'administration du LPS. Les échantillons sont centrifugés, le plasma est récupéré et congelé à -80°C. Le TNF-α est mesuré à l'aide de kits commerciaux (R et D, Abingdon, UK).

Dans ce test, des composés représentatifs de l'invention se sont montrés très actifs, en inhibant la synthèse du TNF-α même à doses très faibles.

Grâce à cette activité et à leur faible toxicité, les composés de formule (I) et ses sels ou solvates peuvent bien être utilisés dans le traitement des maladies liées à des troubles immunitaires et inflammatoires. Notamment les composés de formule (I) peuvent être utilisés pour traiter l'athérosclérose, les maladies autoimmunes, les maladies qui entraînent la démyélinisation des neurones (telles que la sclérose en plaques), l'asthme, l'arthrite rhumatoïde, les maladies fibrotiques, la fibrose idiopathique pulmonaire, la fibrose cystique, la glumérulonéphrite, la spondylite rhumatoïde, l'ostéoarthrite, la goutte, la résorption osseuse et cartilagineuse, l'ostéoporose, la maladie de Paget, le myélome multiple, l'uvéorétinite, les chocs septiques, la septicémie, les chocs endotoxiniques, la réaction du greffon contre l'hôte, le rejet des greffes, le syndrome de détresse respiratoire de l'adulte, la silicose, l'asbestose, la sarcoïdose pulmonaire, la maladie de Crohn, la colite ulcérative, la sclérose latérale amyotrophique, la maladie d'Alzheimer, la maladie de Parkinson, le lupus érythémateux disséminé, les chocs hémodynamiques, les pathologies ischémiques (infarctus myocardique, ischémie myocardique, vasospasme coronarien, angine de poitrine, insuffisance cardiaque, attaque cardiaque), les atteintes post ischémiques de reperfusion, la malaria, les infections mycobactériennes, la méningite, la lèpre, les infections virales (HIV, cytomegalovirus, virus de l'herpès), les infections opportunistes liées au Sida, la tuberculose, le psoriasis, la dermatose atopique et de contact, le diabète, la cachexie, le cancer, les dommages liés aux radiations.

Les composés de formule (I) et leurs sels et solvates pharmaceutiquement acceptables sont administré par voie orale ou parentérale, de préférence par voie orale.

Dans les compositions pharmaceutiques de la présente invention par voie orale, le principe actif peut être administré sous formes unitaires d'administration, en mélange avec des supports pharmaceutiques classiques, aux animaux et aux êtres humains pour le traitement des affections susmentionnées. Les formes unitaires d'administration appropriées comprennent par exemple les comprimés éventuellement sécables, les gélules, les poudres, les granules et les solutions ou suspensions orales.

Lorsqu'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'élixir peut contenir l'ingrédient actif conjointement avec un édulcorant, acalorique de préférence, du méthylparaben et du propylparaben comme antiseptiques, ainsi qu'un agent donnant du goût et un colorant approprié.

Les poudres ou les granules dispersibles dans l'eau peuvent contenir l'ingrédient actif en mélange avec des agents de dispersion ou des agents mouillants, ou des agents de mise en suspension, comme la polyvinylpyrrolidone, de même qu'avec des édulcorants ou des correcteurs du goût.

Le principe actif peut être formulé également sous forme de microcapsules, éventuellement avec un ou plusieurs supports ou additifs.

Dans les compositions pharmaceutiques selon la présente invention, le principe actif peut être aussi sous forme de complexe d'inclusion dans des cyclodextrines, leurs éthers ou leurs esters.

La quantité de principe actif à administrer dépend comme toujours du degré d'avancement de la maladie ainsi que de l'âge et du poids du patient. Néanmoins, les doses unitaires comprennent généralement de 0,001 à 100 mg, mieux de 0,01 à 50 mg, de préférence de 0,1 à 20 mg de principe actif, avantageusement de 0,5 à 10 mg.

Selon un autre de ses aspects, la présente invention concerne une association comprenant un composé de formule (I) ou l'un de ses sels ou solvates pharmaceutiquement acceptables, et au moins un composé choisi parmi les agents immunosuppresseurs, tel que l'interféron bêta-1b; l'hormone adrénocorticotrope; les glucocorticoïdes tels que la prednisone ou la méthylprednisolone; les inhibiteurs de l'interleukine-1, le méthatrexate.

Plus particulièrement, l'invention concerne une association comprenant un composé de formule (I), ou l'un de ses sels ou solvates pharmaceutiquement acceptables et au moins un composé choisi parmi le roquinimex (1,2-dihydro-4-hydroxy-N,1-diméthyl-2-oxo-3-quinolinecarboxanilide), le myloran (produit de chez Autoimmune contenant de la myéline bovine), l'antegren (anticorps humain monoclonal de chez Elan/Athena Neurosciences) l'interféron β-1b recombinant.

D'autres associations possibles sont celles constituées par un composé de formule (I) ou l'un de ses sels ou solvates pharmaceutiquement acceptables et un bloqueur des canaux potassiques, tel que par exemple la fampridine (4-aminopyridine).

L'invention concerne aussi l'utilisation d'un composé (I) pour le traitement des maladies liées à des troubles immunitaires et inflammatoires, notamment l'athérosclérose, les maladies autoimmunes, les maladies qui entraînent la démyélinisation des neurones (telles que la sclérose en plaques), l'asthme, l'arthrite rhumatoïde, les maladies fibrotiques, la fibrose idiopathique pulmonaire, la fibrose cystique, la glumérulonéphrite, la spondylite rhumatoïde, l'ostéoarthrite, la goutte, la résorbtion osseuse et cartilagineuse, l'ostéoporose, la maladie de Paget, le myélome multiple, l'uvéorétinite, les chocs septiques, la septicémie, les chocs endotoxiniques, la réaction du greffon contre l'hôte, le rejet des greffes, le syndrome de détresse respiratoire de l'adulte, la silicose, l'asbestose, la sarcoïdose pulmonaire, la maladie de Crohn, la colite ulcérative, la sclérose latérale amyotrophique, la maladie d'Alzheimer, la maladie de Parkinson, le lupus érythémateux disséminé, les chocs hémodynamiques, les pathologies ischémiques (infarctus myocardique, ischémie myocardique, vasospasme coronarien, angine de poitrine, insuffisance cardiaque, l'attaque cardiaque), les atteintes post ischémiques de reperfusion, la malaria, les infections mycobactériennes, la méningite, la lèpre, les infections virales (HIV, cytomegalovirus, virus de l'herpès), les infections opportunistes liées au Sida, la tuberculose, le psoriasis, la dermatose atopique et de contact, le diabète, la cachexie, le cancer, les dommages liés aux radiations, comprenant l'administration d'un composé de formule (I) ou de l'un de ses sels ou solvates pharmaceutiquement acceptable, seul ou en association avec d'autres principes actifs.

Les exemples qui suivent illustrent l'invention.

### EXEMPLE 1

### 5-(2-(4-(3-Trifluorométhylphényl)-1,2,3,6-tétrahydropyrid-1-yl)éthyl)-benzothiophène et son oxalate.

### 1a) 5-(2-(4-Hydroxy-4-(3-trifluorométhylphényl)pipéridino)éthyl)-benzothiophène

On agite à la température ambiante pendant 3 heures un mélange de 1,47 g (0,006 mole) de 4-hydroxy-4-(3-trifluorométhylphényl)pipéridine, 30 ml de chlorure de méthylène 2,25 ml (0,0162 mole) de triéthylamine, 1,15 g (0,006 mole) d'acide benzothiophène-5-acétique (préparé selon la méthode décrite dans J. Med. Chem. 1997, 40(7):1049-72) et 2,7 g (0,006 mole) de BOP. On ajoute au mélange de l'acétate d'éthyle on lave avec l'acide chlorhydrique 1N, ensuite avec de l'eau, avec une solution de NaHCO₃ et encore avec de l'eau. On sèche la phase organique sur du sulfate de sodium, on filtre et on évapore le solvant. On chauffe au reflux pendant 5 heures le produit brut ainsi obtenu, dissout dans 20 ml de tétrahydrofurane (THF) anhydre et on y ajoute 1,1 ml d'une solution de BH₃Me₂S et 15 ml de THF anhydre. On refroidit jusqu'à 0°C et on y ajoute goutte à goutte 20 ml de méthanol. On chauffe pendant 30 minutes au reflux, on évapore le méthanol et on reprend le résidu dans un mélange NH₄OH / acétate d'éthyle =1/1. On sèche la phase organique sur du sulfate de sodium, on filtre et on évapore le solvant. On purifie le brut de réaction par chromatographie sur colonne de gel de silice en éluant par de l'acétate d'éthyle. On obtient 0,9 g du produit du titre.

### 1b) 5-(2-(4-(3-Trifluorométhylphényl)-1,2,3,6-tétrahydropyrid-1-yl)éthyl)-benzothiophène et son oxalate.

On chauffe 0,8 g (0,0042 mole) d'acide paratoluènsulfonique et 45 ml de chlorobenzène et on distille 25 ml de solvant. On prépare un appareil Markusson en atmosphère d'azote et on ajoute au mélange, après refroidissement, 0,9 g (0,0022 mole) du produit de l'étape précédente dissout dans 25 ml de chlorobenzène. On chauffe au reflux pendant 2 heures et on verse dans une solution saturée en NaHCO₃. On sépare les deux phases, on sèche la phase organique sur du sulfate de sodium, on filtre et on évapore le solvant. On obtient le composé du titre (base) et on prépare son sel oxalate à l'aide d'une solution d'isopropanol saturé en acide oxalique.
P.F. (oxalate) 195-197°C.

### EXEMPLE 2

### 5-(2-(4-(3-Trifluorométhylphényl)-1,2,3,6-tétrahydropyrid-1-yl)éthyl)-benzofurane et son oxalate.

### 2a) Acide benzofurane-5-acétique

On mélange 5 g (0,033 mole) d'acide 4-hydroxyphénylacétique dans 30 ml de DMSO (diméthylsulfoxyde) et on y ajoute 5,7 ml d'une solution aqueuse de NaOH à 50%. Après 10 minutes à la température ambiante on y ajoute 5,16 ml (0,033 mole) de bromoacétaldéhyde diméthylacétal et on chauffe à 110 °C pendant 4 heures. On verse dans de l'acide chlorhydrique 1N et on extrait avec du diéthyléther, on sèche la phase organique sur du sulfate de sodium, on filtre et on évapore le solvant. On mélange 7,85 g (0,029 mole) du produit ainsi obtenu dans 50 ml d'éthanol absolu et on y ajoute 1,5 mmole d'acide p-toluènsulfonique. On chauffe au reflux pendant 3 heures. On évapore l'éthanol on reprend avec une solution de NaHCO₃ à 5% jusqu'à pH neutre, on sèche la phase organique sur du sulfate de sodium, on filtre et on évapore le solvant. On chauffe au reflux 6,57 g d'acide polyphosphorique avec 40 ml de benzène et après 15 minutes on y ajoute 7,06 g du produit obtenu ci-dessus, dissout dans 4 ml de benzène et chauffe au reflux pendant une heure. On élimine le benzène, on lave à l'eau, avec une solution saturée en NaHCO₃ et avec une solution saline. On obtient le produit du titre qui est purifié par flash-chromatographie en éluant par un mélange hexane/acétate d'éthyle=9/1. On obtient ainsi 0.93 g du produit du titre.

### 2b) 5-(2-(4-(3-Trifluorométhylphényl)-1,2,3,6-tétrahydropyrid-1-yl)éthyl)-benzofurane et son oxalate.

En opérant comme décrit dans l'exemple 1a) et 1b) mais en utilisant le produit de l'étape 2a) au lieu de l'acide benzothiophène-5-acétique, on obtient le composé du titre.
P.F. (oxalate) 189-191°C.

### EXEMPLE 3

### 6-(2-(4-(3-Trifluorométhylphényl)-1,2,3,6-tétrahydropyrid-1-yl)éthyl)-benzofurane et son oxalate.

### 3a) Acide benzofurane-6-acétique

En opérant comme décrit dans l'exemple 2a) mais en utilisant l'acide 3-hydroxyphénylacétique au lieu de l'acide 4-hydroxyphénylacétique on obtient le composé du titre.

3b) 6-(2-(4-(3-Trifluorométhylphényl)-1,2,3,6-tétrahydropyrid-1-yl)éthyl)-benzofurane et son oxalate.

En opérant comme décrit dans l'exemple 1a) et 1b) mais en utilisant le produit de l'étape 3a) au lieu de l'acide benzothiophène-5-acétique, on obtient le composé du titre.
P.F. (oxalate) 179-181°C.

### EXEMPLE 4

### 2,3-Diméthyl-5-(2-(4-(3-trifluorométhylphényl)-1,2,3,6-tétrahydropyrid-1-yl)éthyl)-benzofurane et son oxalate.

### 4a) Acide 2,3-diméthylbenzofurane-5-acétique

On mélange 5 g d'acide 4-hydroxyphénylacétique (0,033 mole) dans 100 ml d'éthanol absolu et on y ajoute 1,6 mmole d'acide p-toluènsulfonique. On chauffe au reflux pendant 3 heures. On évapore l'éthanol on reprend avec de l'éther éthylique et on lave avec une solution de NaHCO₃ à 5% jusqu'à pH neutre, on sèche la phase organique sur du sulfate de sodium, on filtre et on évapore le solvant. On chauffe au reflux 6 g (0,033 mole) du produit ainsi obtenu dans 150 ml d'acétone anhydre. On y ajoute 4,45 g (0,033 mole) de bromure de crotyle, 9,33 g de carbonate de potassium et 480 mg d'iodure de sodium et on chauffe au reflux pendant 12 heures. On filtre le carbonate de potassium, on évapore l'acétone on reprend avec de l'éther éthylique et on lave à l'eau. On évapore le solvant et on chauffe le produit à 220°C sans solvant pendant 30 minutes. On dissout 500 mg du produit ainsi obtenu dans 20 ml de chlorure de méthylène et on y ajoute 0,548 g d'iode et 0,124 microl de SnCl₄. On agite pendant 3 heures, on verse dans un mélange eau/glace et on neutralise avec une solution de NaOH 0,5 N. On lave la phase organique avec une solution aqueuse de bisulfite de sodium à 5% et ensuite avec de l'eau. On chauffe au reflux pendant 24 heures 0,37 g du produit ainsi obtenu avec 150 ml de méthanol et 0,43 g de NaOH. On évapore le solvant, on ajoute de l'acide chlorhydrique 1N jusqu'à pH acide et on extrait à l'acétate d'éthyle. On obtient le produit du titre qui est purifié par flash-chromatographie en éluant par un mélange acétate d'éthyle / hexane = 7/3.

### 4b) 2,3-Diméthyl-5-(2-(4-(3-trifluorométhylphényl)-1,2,3,6-tétrahydropyrid-1-yl)éthyl)-benzofurane et son oxalate.

En opérant comme décrit dans l'exemple 1a) et 1b) mais en utilisant le produit de l'étape 4a) au lieu de l'acide benzothiophène-5-acétique, on obtient le composé du titre.
P.F. (oxalate) 206-208°C.

### EXEMPLE 5

### 2-Méthyl-5-(2-(4-(3-trifluorométhylphényl)-1,2,3,6-tétrahydropyrid-1-yl)éthyl)-benzofurane et son oxalate.

### 5a) Acide 2-méthylbenzofurane-5-acétique

En opérant comme décrit dans l'exemple 4a) mais en utilisant le bromure d'allyle au lieu du bromure de crotyle, on obtient le composé du titre.

### 5b) 2-Méthyl-5-(2-(4-(3-trifluorométhylphényl)-1,2,3,6-tétrahydropyrid-1-yl)éthyl)-benzofurane et son oxalate.

En opérant comme décrit dans l'exemple 1a) et 1b) mais en utilisant le produit de l'étape 5a) au lieu de l'acide benzothiophène-5-acétique, on obtient le composé du titre.
P.F. (oxalate) 161-163°C.

### EXEMPLE 6

### 3-Méthyl-5-(2-(4-(3-trifluorométhylphényl)-1,2,3,6-tétrahydropyrid-1-yl)éthyl) benzofurane et son oxalate.

### 6a/ Acide (3-méthylbenzofuran-5-yl)acétique

On chauffe au reflux pendant 5 heures 5 g (0,027 mole) de 4-hydroxyphénylacétate d'éthyle, 120 ml d'acétone, 7,8 g de K₂CO₃, 400 mg d'iodure de sodium et 2,19 ml de chloroacétone. On filtre le K₂CO₃, on évapore le solvant on reprend à l'éther éthylique et on lave à l'eau. On mélange 3,64 g du produit obtenu dans 25 ml de 2,2-diméthoxypropane et on y ajoute 0,4 mmole d'acide p-toluènsulfonique. On chauffe au reflux pendant 3 heures. On y ajoute de l'acétate d'éthyle, on lave avec une solution de bicarbonate de sodium à 5% et ensuite avec une solution saline. On chauffe au reflux 4,2 g d'acide polyphosphorique avec 24 ml de benzène et après 15 minutes on y ajoute 3,66 g du produit obtenu ci-dessus, dissout dans 4 ml de benzène et chauffe au reflux pendant une heure. On élimine le benzène, on lave à l'eau, avec une solution saturée en NaHCO₃ et ensuite avec une solution saline. On purifie le produit par flash-chromatographie en éluant par un mélange hexane/acétate d'éthyle=9/1. On mélange le produit ainsi obtenu à une solution de 0,22 g de KOH en 5,5 ml de méthanol et on chauffe à 80 °C pendant 2 heures. On évapore le solvant et on reprend le résidu dans une solution d'acide chlorhydrique 1N et on extrait au chlorure de méthylène. On sépare les deux phases, on sèche la phase organique sur du sulfate de sodium, on filtre et on évapore le solvant. On obtient le composé du titre.

### 6b) 3-Méthyl-5-(2-(4-(3-trifluorométhylphényl)-1,2,3,6-tétrahydropyrid-1-yl)éthyl)-benzofurane et son oxalate.

En opérant comme décrit dans l'exemple 1a) et 1b) mais en utilisant le produit de l'étape 6a) au lieu de l'acide benzothiophène-5-acétique, on obtient le composé du titre.
P.F. (oxalate) 197-198°C.

### EXEMPLE 7

### 6-(2-(4-(3-trifluorométhylphényl)-1,2,3,6-tétrahydropyrid-1-yl)éthyl)-benzothiophène et son oxalate.

En opérant comme décrit dans l'exemple 1a) et 1b) mais en utilisant l'acide benzothiophène-6-acétique au lieu de l'acide benzothiophène-5-acétique, on obtient le composé du titre.
P.F. (oxalate) 200-201°C.

### EXEMPLE 8

### 2,3-Diméthyl-6-(2-(4-(3-trifluorométhylphényl)-1,2,3,6-tétrahydropyrid-1-yl)éthyl)-benzofurane et son oxalate.

En opérant comme décrit dans l'exemple 1a) et 1b) mais en utilisant l'acide 2,3-diméthylbenzofurane-5-acétique au lieu de l'acide benzothiophène-5-acétique, on obtient le composé du titre.
P.F. (oxalate) 196-198°C.

### EXEMPLE 9

### 2,3-Diméthyl-6-(2-(4-(3-trifluorométhylphényl)-1,2,3,6-tétrahydropyrid-1-yl)éthyl)-benzothiophène et son oxalate.

En opérant comme décrit dans l'exemple 1a) et 1b) mais en utilisant l'acide 2,3-diméthylbenzothiophène-5-acétique au lieu de l'acide benzothiophène-5-acétique, on obtient le composé du titre.
P.F. (oxalate) 194-196°C.

### EXEMPLE 10

### 2,3-Diméthyl-5-(2-(4-(3-fluorophényl)-1,2,3,6-tétrahydropyrid-1-yl)éthyl)-benzofurane et son oxalate.

### 10a) méthanesulfonate de 2-(2,3-diméthyl-benzofuran-5-yl)éthyle.

A une suspension de 3,06 g d'hydrure de lithium aluminium dans 30 ml de THF, sous azote, on ajoute une solution de 6,3 g d'acide 2,3-diméthylbenzofurane-5-acétique dans 125 ml de THF. On chauffe au reflux pendant 4 heures puis on ajoute au mélange réactionnel 100 ml d'eau. On filtre le sel et on évapore le filtrat sous pression réduite pour obtenir une huile. On dissout dans 35 ml de chlorure de méthylène, on ajoute 3,5 ml de triéthylamine et on refroidit à 0-5°C. On ajoute 1,4 ml de chlorure de mésyle et on agite à température ambiante pendant 2 heures. On lave à l'eau, on sèche et on évapore le solvant sous pression réduite. On purifie par chromatographie en éluant par un mélange cyclohexane / acétate d'éthyle = 8/2 et on obtient le produit du titre.

### 10b) 2,3-Diméthyl-5-(2-(4-(3-fluorophényl)-1,2,3,6-tétrahydropyrid-1-yl)éthyl)-benzofurane et son oxalate.

On dissout 0,65 g (2,4 mmole) de produit décrit dans l'étape précédente dans 20 ml de butanol. On y ajoute 0,52 g (2,4 mmole de 4-(3-fluorophényl)-1,2,3,6-tétrahydropyridine et 0,34 g (2,4 mmole) de carbonate de potassium et on chauffe au reflux pendant 5 heures. On évapore le solvant et on lave le résidu à l'eau. On extrait au chlorure de méthylène, on sèche la phase organique et on évapore le solvant sous pression réduite. On purifie le résidu sur colonne de gel de silice en éluant par un mélange cyclohexane / acétate d'éthyle = 8/2 et on obtient le produit du titre. ON prépare l'oxalate à l'aide d'acétone saturée en acide oxalique.
P.F. (oxalate) 203-205°C.

### EXEMPLE 11

### 2,3-Diméthyl-6-(2-(4-(6-chlororopyrid-2-yl)-1,2,3,6-tétrahydropyrid-1-yl)éthyl)-benzofurane et son oxalate.

### 11a) méthanesulfonate de 2-(2,3-diméthyl-benzofuran-6-yl)éthyle.

En opérant comme dans l'exemple 10a) mais en utilisant l'acide 2,3-diméthylbenzofurane-6-acétique au lieu de l'acide 2,3-diméthylbenzofurane-5-acétique, on obtient le composé du titre.

### 11b) 2,3-Diméthyl-6-(2-(4-(6-chlororopyrid-2-yl)-1,2,3,6-tétrahydropyrid-1-yl)éthyl)-benzofurane et son oxalate.

En opérant comme dans l'exemple 10b) mais en utilisant le produit de l'étape précédente au lieu du produit obtenu dans 10a) et la 4-(6-chloropynid-2-yl)-1,2,3,6-tétrahydropyridine au lieu de la 4-(3-fluorophényl)-1,2,3,6-tétrahydropyridine, on obtient le composé du titre.
P.F. (oxalate) 199-200°C.

### EXEMPLE 12

### 2,3-Diméthyl-6-(2-(4-(3-trifluorométhylphényl)-1,2,3,6-tétrahydropyrid-1-yl)éthyl)-benzofurane N-oxyde.

A une solution de 0,2 g (0,5 mmole) de 2,3-Diméthyl-6-(2-(4-(3-trifluorométhylphényl)-1,2,3,6-tétrahydropyrid-1-yl)éthyl)-benzofurane dans 25 ml de chlorure de méthylène à une température de 0-5°C, on ajoute 0,086 g d'acide 3-chloroperbenzoïque. On laisse agiter à 0-5°C pendant 2 heures, on lave avec une solution aqueuse saturée en bicarbonate de sodium et on sépare les deux phases. On sèche la phase organique, on filtre et on évapore sous pression réduite. On purifie par chromatographie en éluant par un mélange méthanol / acétate d'éthyle = 1/1 et on obtient le produit du titre.
P.F. 83-86°C.

## Revendications

1. Composé de formule (I) : 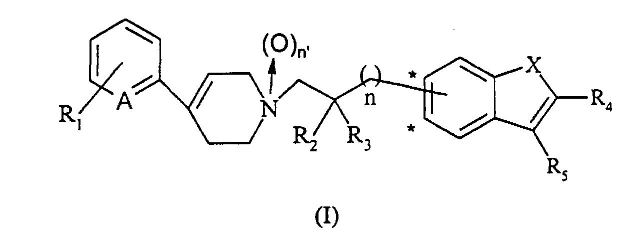 dans laquelle
R₁ représente un atome d'hydrogène ou d'halogène ou un groupe CF₃ ;
R₂ et R₃ représentent indépendamment un atome d'hydrogène ou un groupe méthyle;
n et n' représentent chacun indépendamment 0 ou 1 ;
* représente les positions de rattachement;
A représente N ou CH;
X représente un atome de soufre ou d'oxygène;
R₄ et R₅ représentent indépendamment un atome d'hydrogène ou un groupe (C₁-C₆) alkyle;
ainsi que leurs sels ou solvates.

2. Composé selon la revendication 1 dans lequel n est zéro.

3. Composé selon la revendication 1 dans lequel R₁ est dans la position 3 du benzène.

4. Composé selon la revendication 1 dans lequel R₂ et R₃ sont chacun un atome d'hydrogène.

5. Composé selon la revendication 1 dans lequel R₁ est un groupe CF₃.

6. Composé selon la revendication 1 dans lequel R₄ et R₅ sont chacun un groupe méthyle.

7. Procédé de préparation d'un composé de la revendication 1, **caractérisé en ce que**
(a) on fait réagir le composé de formule (II) : 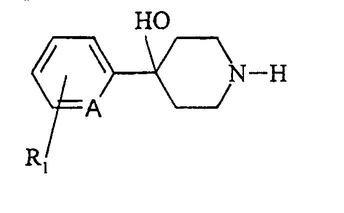 dans laquelle A et R₁ sont définis comme dans la revendication 1, avec un dérivé fonctionnel de l'acide de formule (III) : 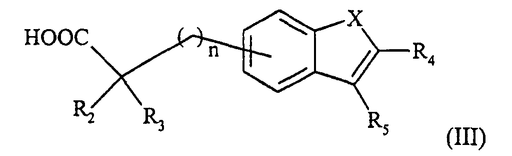 dans laquelle R₂, R₃, R₄,R₅, n et X sont tels que définis dans la revendication 1,
(b) on réduit le groupe carbonyle du composé de formule (IV) : 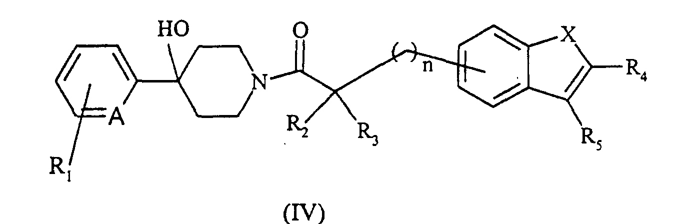
(c) on déshydrate le pipéridinol intermédiaire de formule (V) : 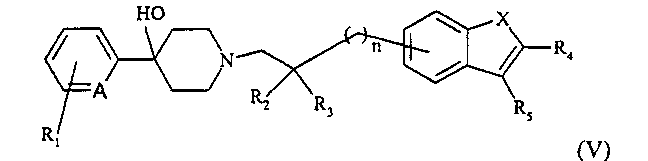
(d) on isole le composé de formule (I) ainsi obtenu et, éventuellement, on le transforme en l'un de ses sels ou solvates, ou son N-oxyde.

8. Procédé de préparation d'un composé de la revendication 1, **caractérisé en ce que** l'on condense une tétrahydropyridine de formule (VI) : 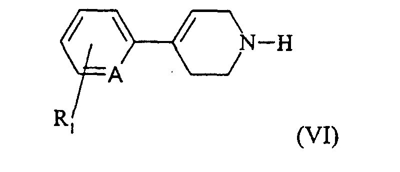 dans laquelle A et R₁ sont tels que définis dans la revendication 1, avec un composé de formule (VII) : 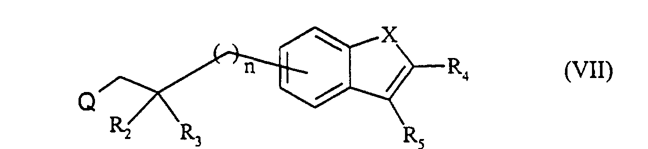 dans laquelle R₂, R₃, R₄, R₅, n et X sont tels que définis ci-dessus et Q est un groupe partant,
on isole le composé de formule (I) ainsi obtenu et le transforme éventuellement en un de ses sels ou solvates, ou en son N-oxyde.

9. Composition pharmaceutique contenant en tant que principe actif un composé de formule (I) selon la revendication 1 ou un de ses sels ou solvates pharmaceutiquement acceptables.

10. Composition selon la revendication 9, **caractérisée en ce qu'**elle contient de 0,01 à 50 mg de principe actif.

11. Utilisation d'un composé de formule (I) selon la revendication 1 ou d'un de ses sels ou solvates pharmaceutiquement acceptables pour la préparation de médicaments destinés au traitement des maladies liées à des troubles immunitaires et inflammatoires, l'athérosclérose, les maladies autoimmunes, les maladies qui entraînent la démyélinisation des neurones, l'asthme, l'arthrite rhumatoïde, les maladies fibrotiques, la fibrose idiopathique pulmonaire, la fibrose cystique, la glumérulonéphrite, la spondylite rhumatoïde, l'ostéoarthrite, la goutte, la résorption osseuse et cartilagineuse, l'ostéoporose, la maladie de Paget, le myélome multiple, l'uvéorétinite, les chocs septiques, la septicémie, les chocs endotoxiniques, la réaction du greffon contre l'hôte, le rejet des greffes, le syndrome de détresse respiratoire de l'adulte, la silicose, l'asbestose, la sarcoïdose pulmonaire, la maladie de Crohn, la colite ulcérative, la sclérose latérale amyotrophique, la maladie d'Alzheimer, la maladie de Parkinson, le lupus érythémateux disséminé, les chocs hémodynamiques, les pathologies ischémiques, les atteintes post ischémiques de reperfusion, la malaria, les infections mycobactériennes, la méningite, la lèpre, les infections virale, les infections opportunistes liées au Sida, la tuberculose, le psoriasis, la dermatose atopique et de contact, le diabète, la cachexie, le cancer, les dommages liés aux radiations.

## Patentansprüche

1. Verbindung der Formel (I): 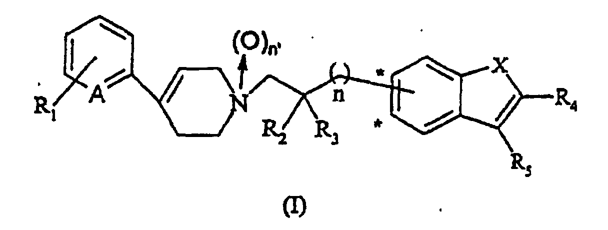 in der
R₁ ein Wasserstoffatom, ein Halogenatom oder eine Gruppe CF₃ bedeutet;
R₂ und R₃ unabhängig voneinander ein Wasserstoffatom oder eine Methylgruppe bedeuten;
n und n' jeweils unabhängig voneinander 0 oder 1 bedeuten;
* die Bindungsstellen markiert;
A N oder CH bedeutet;
X ein Schwefel- oder Sauerstoffatom darstellt;
R₄ und R₅ unabhängig voneinander ein Wasserstoffatom oder eine (C₁-C₆)-Alkygruppe bedeuten;
sowie deren Salze oder Solvate.

2. Verbindung nach Anspruch 1, worin n null bedeutet.

3. Verbindung nach Anspruch 1, worin R₁ in der Position 3 des Benzolkerns steht.

4. Verbindung nach Anspruch 1, worin R₂ und R₃ jeweils ein Wasserstoffatom bedeuten.

5. Verbindung nach Anspruch 1, worin R₁ eine Gruppe CF₃ darstellt.

6. Verbindung nach Anspruch 1, worin R₄ und R₅ jeweils eine Methylgruppe bedeuten.

7. Verfahren zur Herstellung einer Verbindung nach Anspruch 1, **dadurch gekennzeichnet, daß** man
(a) die Verbindung der Formel (II): 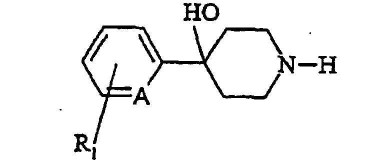 in der A und R₁ die in Anspruch 1 angegebenen Bedeutungen besitzen, mit einem funktionellen Derivat der Säure der Formel (III): 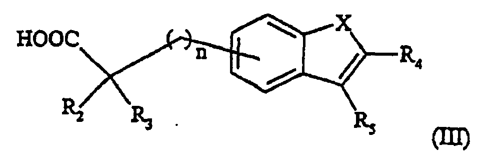 in der R₂, R₃, R₄, R₅, n und X die Anspruch 1 angegebenen Bedeutungen besitzen, umsetzt,
(b) die Carbonylgruppe der Verbindung der Formel (IV) reduziert: 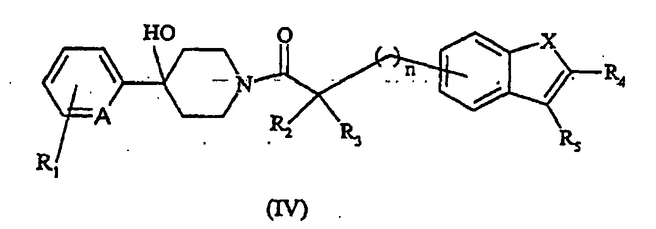
(c) das als Zwischenprodukt anfallende Piperidinol der Formel (V) deshydratisiert: 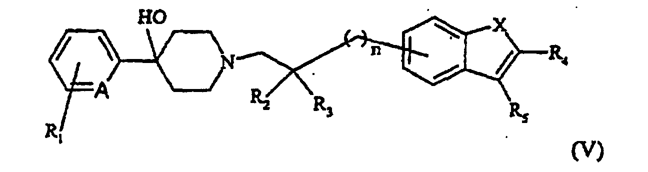
(d) die in dieser Weise erhaltene Verbindung der Formel (I) isoliert und gegebenenfalls in eines ihrer Salze oder Solvate oder ihr N-Oxid umwandelt.

8. Verfahren zur Herstellung einer Verbindung nach Anspruch 1, **dadurch gekennzeichnet, daß** man ein Tetrahydropyridin der Formel (VI): 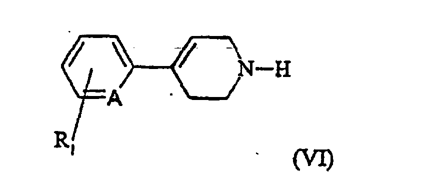 in der A und R₁ die in Anspruch 1 angegebenen Bedeutungen besitzen, mit einer Verbindung der Formel (VII) 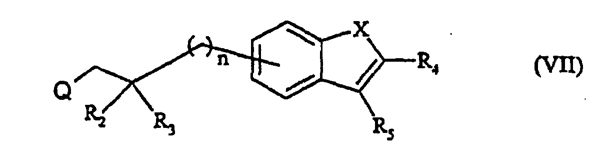 in der R₂, R₃, R₄, R₅, n und X die oben angegebenen Bedeutungen besitzen und Q eine austretende Gruppe darstellt, kondensiert,
die in dieser Weise erhaltenen Verbindung der Formel (I) isoliert und gegebenenfalls in eines ihrer Salze oder Solvate oder in ihr N-Oxid umwandelt.

9. Pharmazeutische Zubereitung, enthaltend als Wirkstoff eine Verbindung der Formel (I) nach Anspruch 1 oder eines ihrer pharmazeutisch annehmbaren Salze oder Solvate.

10. Zubereitung nach Anspruch 9, **dadurch gekennzeichnet, daß** sie 0,01 bis 50 mg des Wirkstoffes enthält.

11. Verwendung einer Verbindung der Formel (I) nach Anspruch 1 oder eines ihrer pharmazeutisch annehmbaren Salze oder Solvate für die Herstellung von Arzneimitteln für die Behandlung von Erkrankungen, die mit Störungen des Immunsystems und Entzündungen verknüpft sind, von Arterosklerose, von Autoimmunerkrankungen, von Erkrankungen, die zu einer Demyelinisation der Neuronen führen, Asthma, rheumatoider Arthritis, fibrotischen Erkrankungen, idiopathischer Lungenfibrose, Mukoviszidose, Glomerulonephritis, rheumatoider Spondylitis, Osteoarthritis, Gicht, der Knochen- und Knorpel-Resorption, Osteoporose, der Paget-Kranhkeit, multiplen Myelomen, Uveoretinitis, septischen Schocks, Blutvergiftung, endotoxinischen Schocks, Reaktion von Transplantaten gegen den Wirt, Abstoßung von Transplantaten, dem Atemnotsyndrom beim Erwachsenen, Silikose, Asbestose, Lungensarkoidose, der Crohnschen Krankheit, der ulzerativen Colitis, der amyotrophen Lateralsklerose, der Alzheimerschen Krankheit, der Parkinsonschen Krankheit, des ansteckenden Lupus erythematodes, von hämodynamischen Schocks, von ischämischen Krankheiten, von postischämischen Schlaganfällen der Reperfusion, Malaria, mykobakteriellen Infektionen, Meningitis, Lepra, Virusinfektionen, mit AIDS verknüpften opportunistischen Infektion, Tuberculose, Psoriasis, atopische und Kontakt-Dermatose. Diabetes, Kachexie, Krebs und Strahlenschäden.

## Claims

1. Compound of formula (I): 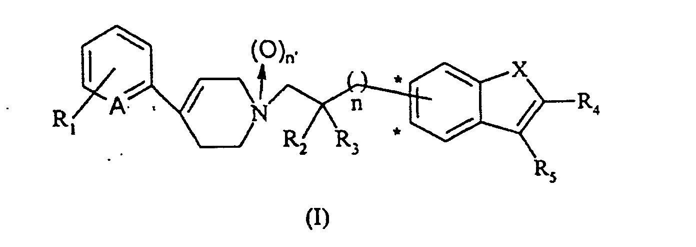 in which
R₁ represents a hydrogen or halogen atom, or a group CF₃;
R₂ and R₃ represent, independently, a hydrogen atom or a methyl group;
n and n' each represent, independently, 0 or 1;
* represents the positions of attachment;
A represents N or CH;
X represents a sulphur or oxygen atom;
R₄ and R₅ represent, independently, a hydrogen atom or a (C₁-C₆) alkyl group;
and their salts or solvates.

2. Compound according to Claim 1, in which n is zero.

3. Compound according to Claim 1, in which R₁ is in position 3 of the benzene.

4. Compound according to Claim 1, in which R₂ and R₃ are each a hydrogen atom.

5. Compound according to Claim 1, in which R₁ is a group CF₃.

6. Compound according to Claim 1, in which R₄ and R₅ are each a methyl group.

7. Method for preparing a compound of Claim 1, **characterized in that**
(a) the compound of formula (II): 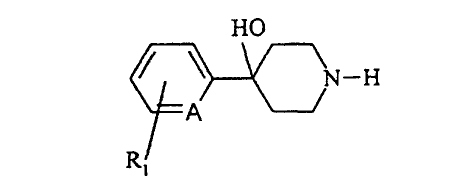 in which A and R₁ are defined as in Claim 1, is reacted with a functional derivative of the acid of formula (III): 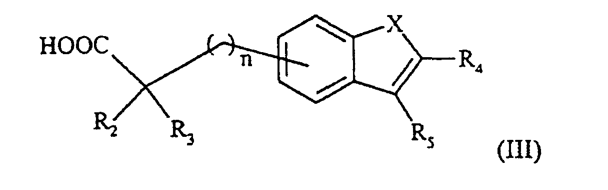 in which R₂, R₃, R₄, R₅, n and X are as defined in Claim 1,
(b) the carbonyl group of the compound of formula (IV) : 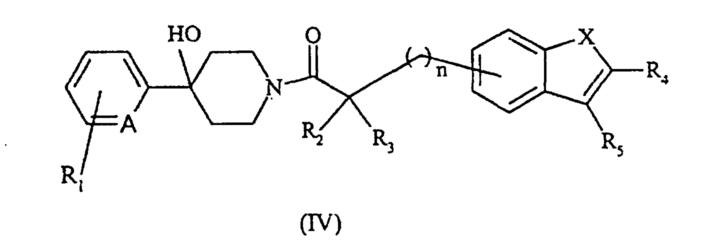 is reduced,
(c) the intermediate piperidinol of formula (V): 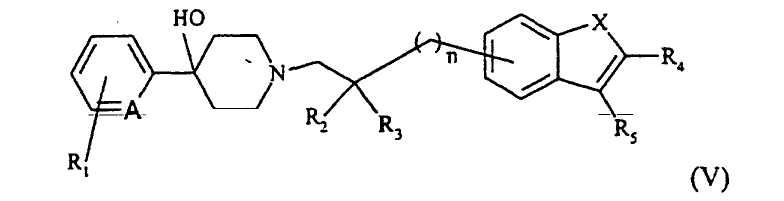 is dehydrated,
(d) the compound of formula (I) thus obtained is isolated and, optionally, it is transformed into one of its salts or solvates, or its N-oxide.

8. Method for preparing a compound of Claim 1, **characterized in that** a tetrahydropyridine of formula (VI) : 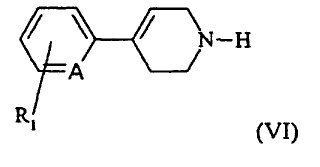 in which A and R₁ are as defined in Claim 1, is condensed with a compound of formula (VII): 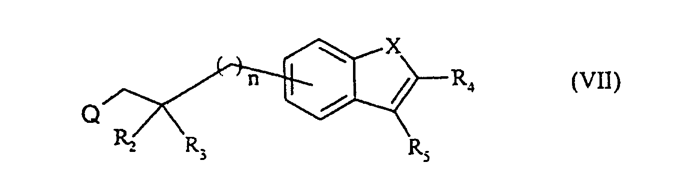 in which R₂, R₃, R₄, R₅, n and X are as defined above and Q is a leaving group,
the compound of formula (I) thus obtained is isolated and is optionally transformed into one of its salts or solvates, or into its N-oxide.

9. Pharmaceutical composition containing, as active principle, a compound of formula (I) according to Claim 1, or one of its pharmaceutically acceptable salts or solvates.

10. Composition according to Claim 9, **characterized in that** it contains from 0.01 to 50 mg of active principle.

11. Use of a compound of formula (I) according to Claim 1, or of one of its pharmaceutically acceptable salts or solvates, for preparing medicinal products intended for the treatment of diseases related to immune and inflammatory disorders, atherosclerosis, autoimmune diseases, diseases which lead to the demyelinization of neurons, asthma, rheumatoid arthritis, fibrotic diseases, idiopathic pulmonary fibrosis, cystic fibrosis, glumerulonephritis, rheumatoid spondylitis, osteoarthritis, gout, bone and cartilage resorption, osteoporosis, Paget's disease, multiple myeloma, uveoretinitis, septic shock, . septicemia, endotoxic shock, graft-versus-host reaction, graft rejection, adult respiratory distress syndrome, silicosis, asbestosis, pulmonary sarcoidosis, Crohn's disease, ulcerative colitis, amyotrophic lateral sclerosis, Alzheimer's disease, Parkinson's disease, lupus erythematosus disseminatus, hemodynamic shock, ischemic pathological conditions, postischemic reperfusion injuries, malaria, mycobacterial infections, meningitis, leprosy, viral infections, AIDS-related opportunistic infections, tuberculosis, psoriasis, atopic and contact dermatosis, diabetes, cachexia, cancer and radiation-mediated damage.
